# FASCICULE DE BREVET EUROPEEN

(11) **EP 0 963 763 B1**
(45) Date de publication et mention de la délivrance du brevet: **10.12.2003**
(21) Numéro de dépôt: 99401413.2
(22) Date de dépôt: 10.06.1999
(51) Int. Cl.: A61M 5/32

(54) **Protecteur d'aiguille hypodermique**
Schutzvorrichtung für Subkutaninjektionsnadel
Hypodermic needle guard

(30) Priorité: 11.06.1998 FR 9807361
(43) Date de publication de la demande: 15.12.1999
(73) Titulaire: Société VYGON, F-95440 Ecouen (FR)
(72) Inventeur: Huet, Jean-Max, 92110 Clichy (FR)
(74) Mandataire: Schrimpf, Robert

(56) Documents cités:
- EP-A- 0 589 506
- WO-A-90/01348
- WO-A-94/11050
- US-A- 4 950 249
- US-A- 5 702 369

## Description

L'invention concerne un protecteur d'aiguille hypodermique, notamment pour une aiguille épicrânienne.

On connaît un protecteur d'aiguille de seringue hypodermique constitué d'une pièce monobloc venue de moulage en résine de synthèse, cette pièce comportant une partie distale qui forme un capuchon dans lequel est logée l'extrémité distale de l'aiguille, et une partie proximale qui forme une embase dans laquelle est encastrée l'extrémité proximale de l'aiguille, ces deux parties étant articulées l'une à l'autre par une zone amincie constituant une ligne de pliage à effet élastique en sorte qu'une action manuelle permette de faire basculer le capuchon pour démasquer l'aiguille et que le capuchon revienne de lui-même dans sa position de protection après cessation de cette action manuelle, comme décrit dans la publication US A 5 693 022.

Ce type de protecteur présente l'avantage, par rapport aux dispositifs antérieurs nécessitant des ressorts de rappel, que le capuchon revienne de lui-même dans sa position de protection après cessation de l'action manuelle qui l'en avait écarté.

On assure ainsi à moindre frais une bonne sécurité contre les piqûres accidentelles.

Dans les réalisations décrites dans la publication précitée le protecteur comporte sur la partie formant capuchon un doigt de préhension sur lequel l'utilisateur doit agir pour provoquer le basculement du capuchon.

L'utilisateur doit donc d'une part tenir l'embase du protecteur pour manipuler l'aiguille et en même temps agir sur le doigt du capuchon pour dévoiler l'aiguille.

La présente invention a pour but de fournir un protecteur dont la manipulation soit plus aisée.

On y parvient selon l'invention avec un protecteur d'aiguille tel que défini dans la revendication 1.

Dans une réalisation préférée, l'embase du protecteur comporte deux ailettes de préhension aptes à être pivotées manuellement dans un sens pour provoquer le basculement du capuchon.

L'opérateur réalise donc simultanément la tenue en main de l'aiguille et le basculement du capuchon.

On décrira ci-après un mode de réalisation préféré du protecteur de l'invention, en référence aux figures du dessin joint, la description et les figures faisant apparaître d'autres caractéristiques de la présente invention.

Sur les figures :
· La figure 1 est une vue en plan du protecteur dans sa configuration de protection de l'aiguille ;
· La figure 2 est une coupe du protecteur de la figure 1 par un plan contenant l'aiguille ;
· La figure 3 est une coupe du protecteur par un plan perpendiculaire à l'aiguille, dans la région de l'embase ;
· Les figures 4 et 5 sont des vues en perspective du protecteur au cours de deux phases successives de l'opération de dégagement de l'aiguille ;

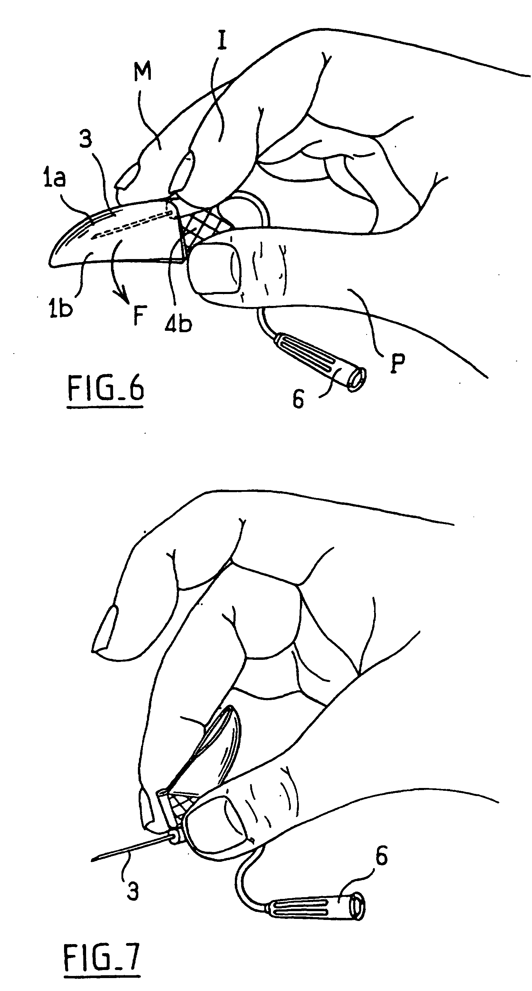

· Les figures 6 et 7 sont d'autres vues en perspective montrant comment un opérateur peut saisir le protecteur et le manipuler avec les doigts d'une seule main ;
· La figure 8 est une perspective montrant cinq phases successives d'une opération de découvrement de l'aiguille pour une ponction ;
· La figure 9 est une perspective montrant la phase initiale de l'opération d'extraction de l'aiguille.

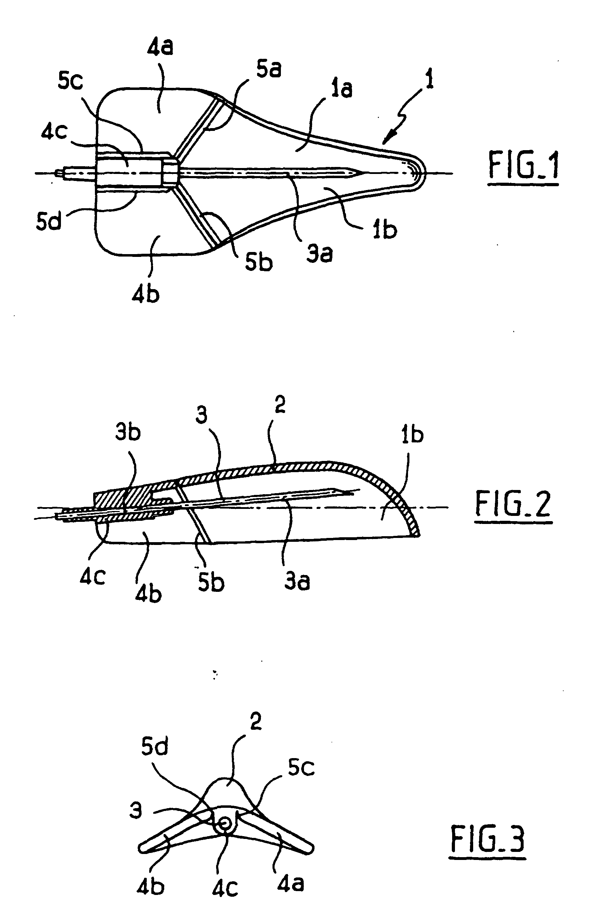

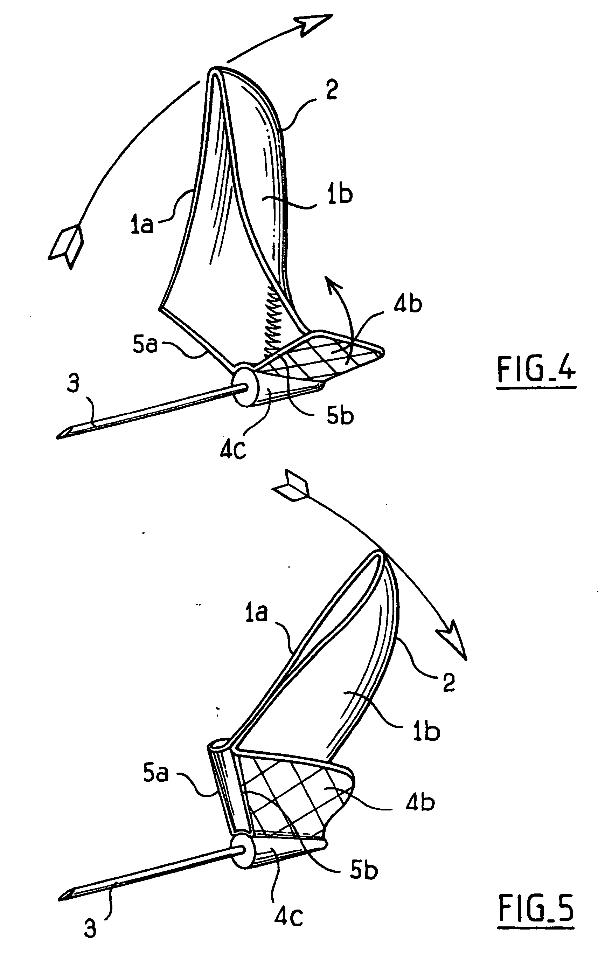

Le protecteur représenté sur les figures comporte un capuchon (1) constitué de deux parois (1a ; 1b) attenantes obliquement le long d'une zone (2) qui longe l'aiguille (3) et qui remonte devant le nez (3a) de l'aiguille, conférant au capuchon une section transversale en forme de « V » comme on le voit sur les figures et notamment sur la figure 4, dont l'angle d'ouverture décroît au fur et à mesure que le plan de cette section se rapproche de l'extrémité du capuchon.

Selon l'invention, le protecteur comporte d'autre part une embase (4) constituée de deux ailettes (4a ; 4b) qui sont articulées latéralement à un moyeu central (4c) dans lequel est encastrée la partie d'extrémité proximale de l'aiguille (3b), ces deux ailettes prolongeant respectivement les deux parois (1a ; 1b) du capuchon auquel elles sont reliées par des zones amincies (5a ; 5b).

Les ailettes sont articulées au moyeu par deux charnières (5c ; 5d) constituées par des parties de paroi amincies.

Le moyeu (4c) est de préférence en majeure partie en saillie par rapport aux ailettes (4a ; 4b), comme on le voit notamment sur la figure 3.

De préférence les parois du capuchon et les ailettes sont des parois minces relativement souples pour ne pas être traumatisantes.

De préférence l'extrémité proximale de l'aiguille traverse le moyeu pour être raccordée à un connecteur 6.

De préférence les ailettes ont une épaisseur de paroi supérieure à celle des parois du capuchon.

Enfin, on notera que dans la réalisation représentée les zones amincies (5a ; 5b) forment un « Y » avec le moyeu (4c).

En service, le protecteur est manipulé avec l'aiguille en position sous le capuchon, le biseau de l'aiguille étant apparent.

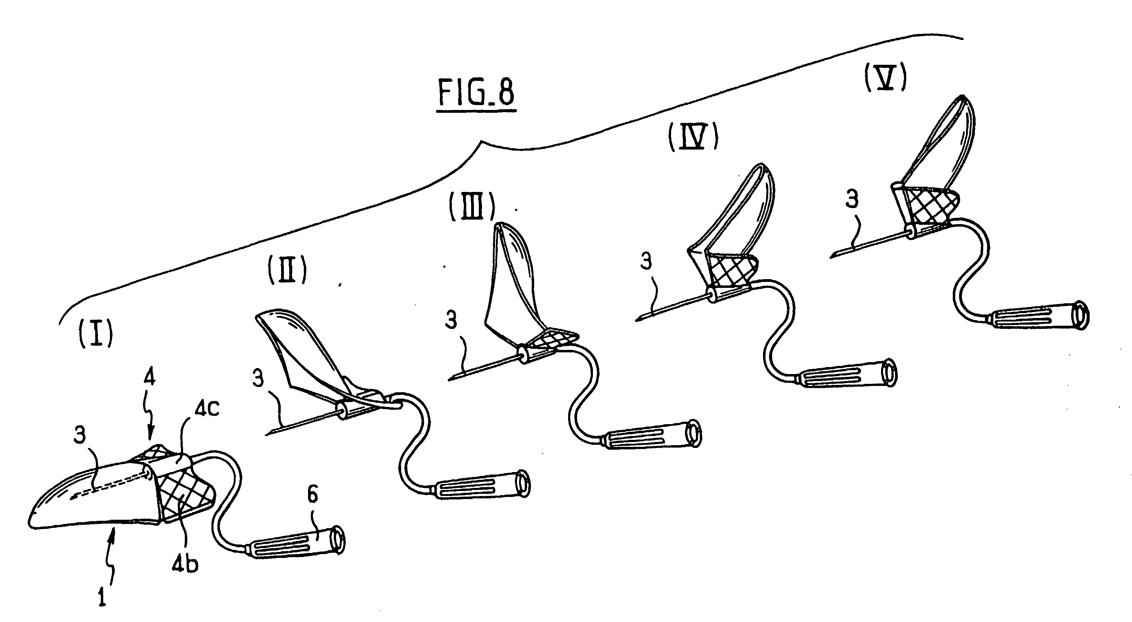

L'opérateur maintient les ailettes respectivement avec le pouce (P) et le majeur (M) tout en exerçant une pression avec l'index (I) sur le moyeu pour les repousser vers le dos du capuchon comme on le voit sur la figure 4, de façon à relever les ailettes, ce qui provoque le basculement du capuchon vers le haut puis vers l'arrière dans le sens de la flèche (F), dégageant progressivement l'aiguille (3) qui se trouve sous le capuchon, comme on le voit sur la figure 8.

On remarque que dans la position où l'àiguille est découverte, prête pour une opération de ponction, l'opérateur tient le dispositif avec essentiellement le pouce et le majeur qui pressent l'une contre l'autre les deux ailettes de l'embase.

Après la ponction, si l'aiguille doit rester en place, les deux ailettes permettent de la fixer au patient par un ruban adhésif, de façon connue en soi.

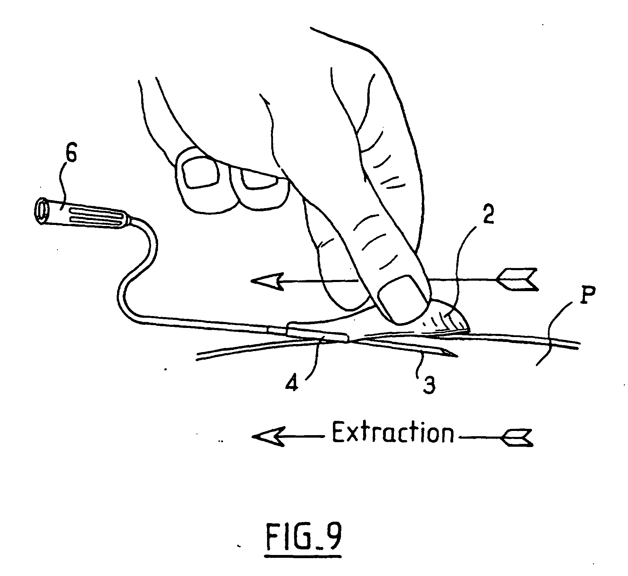

Pour extraire l'aiguille, il n'est pas nécessaire de replier les ailettes qui se trouvent alors à plat sur la peau (P), il suffit de saisir le dispositif par le capuchon (figure 9) et l'aiguille revient automatiquement dans le capuchon dès qu'elle est sortie de la peau.

L'invention n'est pas limitée à la réalisation qui a été décrite.

## Revendications

1. Protecteur d'aiguille hypodermique constitué d'une pièce monobloc venue de moulage en résine de synthèse, cette pièce comportant une partie distale qui forme un capuchon (1) dans lequel est logée la partie d'extrémité distale (3a) de l'aiguille (3), et une partie proximale qui forme une embase (4) dans laquelle est encastrée la partie d'extrémité proximale (3b) de l'aiguille, ces deux parties étant articulées l'une à l'autre par une zone amincie (5a ; 5b) constituant une ligne de pliage à effet élastique en sorte qu'une action manuelle permette de faire basculer le capuchon pour démasquer l'aiguille et que le capuchon revienne de lui-même dans sa position de protection après cessation de cette action manuelle, **caractérisé en ce que** le capuchon (1) est constitué de deux parois (1a ; 1b) attenantes obliquement le long d'une zone (2) qui longe l'aiguille et qui remonte devant le nez de l'aiguille, conférant au capuchon une section transversale en forme de « V » dont l'angle d'ouverture décroît au fur et à mesure que le plan de section se rapproche de l'extrémité du capuchon, **en ce que** l'embase (4) est constituée de deux ailettes (4a ; 4b) qui sont articulées latéralement à un moyeu central (4c) dans lequel est encastrée la partie d'extrémité proximale (3b) de l'aiguille, ces deux ailettes prolongeant respectivement les deux parois du capuchon auxquelles elles sont reliées par ladite zone amincie (5a; 5b), en sorte qu'une action manuelle sur les ailettes visant à les faire pivoter sur le moyeu vers l'arrière du capuchon provoque le basculement du capuchon vers l'arrière le long de la zone amincie, ce qui dégage l'extrémité distale de l'aiguille, et qu'un relâchement de cette action manuelle provoque le retour des ailettes et du capuchon dans leurs positions d'origine.

2. Protecteur selon la revendication 1 dont l'embase (4) comporte deux ailettes de préhension (4a ; 4b) aptes à être pivotées manuellement dans un sens pour faire basculer le capuchon.

3. Protecteur selon la revendication 1 ou 2 dont le moyeu est en majeure partie en saillie par rapport aux ailettes.

4. Protecteur selon l'une des revendications 1 à 3 dont les parois du capuchon sont plus minces que les ailettes.

5. Protecteur selon l'une des revendications 1 à 4 dont le moyeu (4c) est traversé par la partie d'extrémité proximale (3b) de l'aiguille.

6. Protecteur selon l'une des revendications 1 à 5 dont les zones amincies et le moyeu forment un « Y ».

## Claims

1. A hypodermic needle protector constituted by a single piece of molded synthetic resin, said piece having a distal portion which forms a cap (1) in which the distal end portion (3a) of the needle (3) is housed, and a proximal portion forming a base (4) in which the proximal end portion (3b) of the needle is engaged, said two portions being hinged to each other via a thin zone (5a; 5b) constituting an elastic effect fold line such that manual action makes it possible to tilt the cap so as to reveal the needle and such that the cap returns on its own to its protective position after said manual action ceases, the protector being **characterized in that** the cap (1) is constituted by two walls (1a; 1b) meeting obliquely along a zone (2) which runs along the needle and which rises over the tip of the needle, giving the cap a V-shaped cross-section with the opening angle thereof decreasing as the section plane comes closer to the end of the cap, **in that** the base (4) is constituted by two flaps (4a; 4b) which are hinged laterally to a central hub (4c) in which the proximal end portion (3b) of the needle is engaged, said two flaps respectively extending the two walls of the cap to which they are connected via said thin zone (5a; 5b) such that manual action on the flaps to cause them to pivot on the hub towards the back of the cap causes the cap to tilt backwards along the thin zone, thereby disengaging the distal end of the needle, and that releasing said manual action causes the flaps and the cap to return to their original positions.

2. A protector according to claim 1, in which the base (4) has two flaps (4a; 4b) for grasping that are suitable for being pivoted manually in one direction to cause the cap to tilt.

3. A protector according to claim 1 or claim 2, in which the major portion of the hub projects relative to the flaps.

4. A protector according to any one of claims 1 to 3, in which the walls of the cap are thinner than the flaps.

5. A protector according to any one of claims 1 to 4, in which the hub (4c) has the proximal end portion (3b) of the needle passing therethrough.

6. A protector according to any one of claims 1 to 5, in which the thin zones and the hub form a Y-shape.

## Patentansprüche

1. Schutzvorrichtung für eine Nadel zur subkutanen Injektion, gebildet aus einem aus einem Stück bestehenden Teil, welches aus einem Gießen unter Verwendung von Kunstharz hervorgeht, wobei dieses Teil einen distalen Abschnitt, der eine Schutzkappe (1), in welcher sich der distale Endabschnitt (3a) der Nadel (3) befindet, bildet, und einen proximalen Abschnitt, der ein Fußteil (4), in welches der proximale Endabschnitt (3b) der Nadel eingelassen ist, bildet, umfasst, wobei die zwei Abschnitte gelenkig miteinander verbunden sind durch eine abgeschrägte Zone (5a; 5b), welche eine Faltungszone mit elastischer Wirkung bildet derart, dass eine manuelle Einwirkung erlaubt, die Schutzkappe umkippen zu lassen, um die Nadel freizulegen, und dass die Schutzkappe von sich aus nach der Beendigung dieser manuellen Einwirkung in ihre Schutzposition zurückkehrt, **dadurch gekennzeichnet, dass** die Schutzkappe (1) aus zwei Wänden (1a; 1b) gebildet wird, die schräg entlang einer Zone (2), die sich längs der Nadel erstreckt und die vor dem Kopf der Nadel aufsteigt, aneinandergrenzen, was der Schutzkappe einen "V"-förmigen Querschnitt verleiht, dessen Öffnungswinkel in dem Maße abnimmt, wie die Schnittfläche sich dem Ende der Schutzkappe nähert, dass das Fußteil (4) aus zwei Flügeln (4a; 4b) gebildet wird, die gelenkig seitlich mit einer zentralen Nabe (4c), in welche der proximale Endabschnitt (3b) der Nadel eingelassen ist, verbunden sind, wobei diese beiden Flügel die beiden Wände der Schutzkappe, mit denen sie durch die abgeschrägte Zone (5a; 5b) verbunden sind, jeweils verlängern derart, dass eine manuelle Einwirkung auf die Flügel, welche darauf abzielt, diese auf der Nabe in Richtung der Rückseite der Schutzkappe schwenken zu lassen, das Kippen der Schutzkappe in Richtung der Rückseite entlang der abgeschrägten Zone bewirkt, was das distale Ende der Nadel freilegt, und dass eine Beendigung dieser manuellen Betätigung die Rückkehr der Flügel und der Schutzkappe in ihre Ausgangspositionen auslöst.

2. Schutzvorrichtung nach Anspruch 1, deren Fußteil (4) zwei Greifflügel (4a; 4b) umfasst, die dazu geeignet sind, manuell in einer Richtung geschwenkt zu werden, um die Schutzkappe kippen zu lassen.

3. Schutzvorrichtung nach Anspruch 1 oder 2, deren Nabe größtenteils in Bezug auf die Flügel hervorragt.

4. Schutzvorrichtung nach einem der Ansprüche 1 bis 3, bei der die Wände der Schutzkappe dünner als die Flügel sind.

5. Schutzvorrichtung nach einem der Ansprüche 1 bis 4, bei der die Nabe (4c) von dem proximalen Endabschnitt (3b) der Nadel durchdrungen wird.

6. Schutzvorrichtung nach einem der Ansprüche 1 bis 5, bei der die abgeschrägten Zonen und die Nabe ein "Y" bilden.
